# EUROPEAN PATENT SPECIFICATION

(11) **EP 1 201 766 B1**
(45) Date of publication and mention of the grant of the patent: **07.09.2005**
(21) Application number: 00944051.2
(22) Date of filing: 11.07.2000
(51) Int. Cl.: C12Q 1/68, C12N 15/62

(54) **CELL LINE COMPRISING THE PROMOTER OF CYCLOOXYGENASE-2 (COX-2) AND A WITNESS GENE, AND USE THEREOF IN THE SEARCH OF COX-2 TRANSCRIPTIONAL INDUCTION SELECTIVE INHIBITORS**
ZELLINIE, ENTHALTEND DEN PROMOTOR VON CYCLOOXYGENASE-2 (COX-2) SOWIE EIN ZEUGENGEN, UND VERWENDUNG DERSELBEN IN DER SUCHE VON SELEKTIVEN INDUKTOREN EINER COX-2 TRANSKRIPTIONELLEN INDUKTION
LIGNEE CELLULAIRE COMPRENANT LE PROMOTEUR DE LA CYCLOOXYGENASE-2 (COX-2) ET GENE TEMOIN, LEUR UTILISATION DANS LA RECHERCHE D'INHIBITEURS SELECTIFS DE L'INDUCTION DE LA TRANSCRIPTION DE COX-2

(30) Priority: 12.07.1999 ES 9901557
(43) Date of publication of application: 02.05.2002
(73) Proprietor: LABORATORIOS DEL DR. ESTEVE, S.A., 08041 Barcelona (ES)
(72) Inventor: FRESNO ESCUDERO, M., Cent. Bio. Mole. Severo Ochoa, E-28049 Madrid (ES); INIGUEZ PENA, M.A., Centro Bio. Mole. Secero Ochoa, Cantoblanco, E-28049 Madrid (ES)
(74) Representative: Carpintero Lopez, Francisco
(86) International application number: PCT/ES2000/000245
(87) International publication number: WO 2001/004350

(56) References cited:
- WO-A-98/37235
- US-A- 5 445 941
- US-A- 5 556 754
- US-A- 5 569 588
- JAOU-CHEM H. ET AL.: 'Gene transfer to cultured human endometrial stromal cells: A model to study cyclooxygenase-2 gene regulations' FERTILITY AND STERILITY vol. 70, no. 4, 1998, pages 734 - 739, XP000996127
- HIROYASU INOUE ET AL.: 'Transcriptional role of nuclear factor KB site in the induction by lipopolysaccharide and suppression by dexamethasone of cyclooxygenase-2 in U937 cell' BIOCHEMICAL AND BIOPHYSICAL RESEARCH COMMUNICATIONS vol. 244, 1998, pages 143 - 148, XP000986132
- BLANCO, J.C.G. ET AL: J. EXP. MED., vol. 191, 2000, pages 2131-2144,

## Description

### FIELD OF THE INVENTION

This invention relates, in general, to the search for products with potential therapeutic applications. In particular, the invention relates to a method for the search for compounds that selectively inhibit the induction at a transcriptional level of cyclooxygenase-2 that comprises the use of a cell line that expresses in a stable manner a construct of DNA in which the gene promoter sequence of cyclooxygenase-2 controls the expression of a reporter gene in response to appropriate stimuli.

### BACKGROUND OF THE INVENTION

The cyclooxygenase (cox) is an enzyme implicated in numerous processes. Two isoforms of cox are known, cyclooxygenase 1 (cox-1) and cyclooxygenase 2 (cox-2). Although both isoforms are related to the production of prostaglandins implicated in physiological processes, it seems that cox-2 is the isoform predominately implicated in various pathologies such as inflammation, carcinogenesis, angiogenesis and certain neurodegenerative processes.

Induction at a transcriptional level of cox-2 occurs in response to several factors, among which can be found the expression of oncogenes, the treatment of tumours with promoters, mytogens, pro-inflammatory stimuli, growth factors and cytokines [reviewed by Smith and DeWitt, 1996; Griswold and Adams, 1996; Jouzeau et al., 1997 (see section relating to REFERENCES)]. In most cases, the induction of this enzyme translates into an increase in the synthesis of prostaglandins, although other modes of action cannot be discarded.

The capacity of certain drugs of the family of nonsteroidal anti-inflammatory drugs (NSAIDs) to inhibit cox-2 explains their therapeutic effects [reviewed by Smith and DeWitt, 1996; Griswold and Adams, 1996; Jouzeau et al., 1997]. Similarly, there is growing evidence that the inhibition of cox-2 both by NSAIDS and by glucocorticoids or by cyclosporin A has immunosuppressive effects [Iñiguez et al., 1998; Hall and Wolf, 1997; Zhou et al., 1994; and the reviews cited earlier]. Other actions of induction of cox-2 relate to the implication of this enzyme in cancer, angiogenesis and neurodegenerative processes such as Alzheimer's disease. It has been found that both inhibition of the induction at a transcription level of cox-2 and the enzymatic inhibition of cox-2 attenuate these processes [Shiff et al., 1996; Tsujii et al., 1997 y 1998; Subbaramiah et al., 1998; Pasinetti, 1998].

After discovering the inducible cox-2 isoform of the cyclooygenase enzyme, the methods for identification of new anti-inflammatory drugs have focussed on selecting compounds that are selective inhibitors of the enzymatic activity of cox-2 against the constitutive isoform cox-1. There are several types of system for this. Some use *in vitro* assays with purified or semi-purified cox-2 [Famaey, 1997; Noreen et al., 1998]. Other authors use animal or human cell lines that predominantly express the cox-2 isoforms in natural conditions or after induction with stimuli [Famaey, 1997; Berg et al., 1997]. Some use cell lines of animal or human origin in which the cox-2 protein is over-expressed by means of stable transfection of cDNA coding for this protein [Lora et al., 1997; O'Neill et al., 1995; Cromlish and Kennedy, 1996]. In some cases, it has been possible to determine using mRNA analysis whether such compounds inhibit the induction of cox-2 at a transcriptional level [Tao et al., 1998; Subbaramiah et al., 1998]. Systems have also been established for studying inhibitory compounds by means of *in vivo* assays, either with whole blood or with purified cells from healthy donors [Famaey, 1997; Brideau et al., 1996].

In any case, the main limitation of these systems lies in the fact that they allow selection of compounds that inhibit the enzymatic activity of the cox-2 enzyme, without considering their effects on the induction of the production of the protein, the step prior to production of prostaglandins by this enzyme. In addition to this limitation, it has been shown that the relative potencies of these compounds vary for the same drug for different types of assay. Similarly, an important aspect for consideration concerns the inhibition of the physiological activity of cox-2, which would also be inhibited by the type of compounds identified by the aforementioned systems, which could lead to adverse side effects.

It is known from **WO 98/37235** a method of screening agents as candidates for drugs which suppress induction of COX-2 promoter. This screening procedure relies on the use of a recombinant vector comprising a construct containg 1432 bases from the COX-2 transcription start site (-1432 to +59) ligated to the luciferase gene.

Document **Huang, J-C and M.Y. Dawood (1988) Fertility and Sterility 70:734-739** discloses the determination of the effect of PMA and IL-1β on COX-2 promoter activity based on the measurement of luciferase activity generated in endometirial stromal cells transfected with a vector that contains a luciferase reporter and a 900-base pair promoter sequence (-891 to +9 relative to the transcription site) corresponding to the human gene.

Document **Inoue, H. and T. Tanabe (1998) Biochem. Biophys. Res. Comm. 244: 143-148** studies the transcriptional role of the NF-kB site of the COX-2 gene in U937 cells employing luciferase reporter vector driven by the human COX-2 promoter region (nucleotides -327 to +59) stably transfected into U937 cells.

There is therefore a need to develop a method for searching for compounds that selectively inhibit induction of cox-2 at a transcriptional level by different stimuli that overcomes the drawbacks mentioned above.

### OVERVIEW OF THE INVENTION

This invention provides a solution to the existing need that consists of developing an assay system for the search of compounds that selectively inhibit the induction of cox-2 at a transcriptional level by different stimuli. This criterion allows compounds to be selected that inhibit the production of cox-2, and so will act as inhibitors of the actions derived from an increase in the expression of cox-2 and of the subsequent increase in the production of prostaglandins that set off various pathological processes. Among other processes, the following processes can be highlighted: inflammatory processes, uncontrolled cellular proliferation, angiogenesis, carcinogenesis and neurodegenerative pathologies, as were described earlier. The criterion for the selection of compounds according to this invention lies in the inhibition of the inducible activity of the promoter of cox-2. Therefore, those compounds that inhibit the physiological basal activity of production of cox-2 will not be selected.

For the development of the solution provided by this invention, it has been necessary to construct a cell line that expresses in a stable fashion a construct of DNA in which the promoter sequence of the cox-2 gene controls the expression of the reporter gene in response to a suitable stimulus. The regulation of the expression of the cox-2 gene is determined by the regulatory activity of its promoter, while the measurement of the activity of the reporter gene provides an indirect measure of the activity of the promoter of cox-2 in response to different agents.

Thus, an object of this invention constitutes a DNA construct (or recombinant DNA) that comprises a promoting sequence of the cox-2 gene and a reporter gene, operatively joined to each other, such that said promoter sequence of the gene controls the expression of the reporter gene in response to a suitable stimulus.

An additional object of this invention constitutes a vector, such as a plasmid or an expression vector, that comprises said DNA construct.

Another additional object of this invention constitutes a cell line that contains said DNA construct, or said plasmid that contains said DNA construct, that expresses it in a stable fashion.

Finally, another additional object of this invention constitutes a method for the search for compounds that selectively inhibit the induction of cyclooxygenase-2 at a transcriptional level, that comprises the use of said cell line that expresses said DNA construct in a stable fashion.

### BRIEF DESCRIPTION OF THE FIGURES

Figure 1 shows the sequence of the promoter zone of the cox-2 gene. The arrows indicate the sequences of hybridisation of the oligonucleotides used in the PCR reaction [polymerase chain reaction].
Figure 2 shows the strategy for cloning the promoting region of the cox-2 gene in the pXP2 plasmid in order to obtain the construct prom2-1906-LUC.
Figure 3 shows the results of the analysis by RT-PCR [reverse transcription-polymerase chain reaction] of the expression of mRNA of cox-2 in Jurkat cells. In Figure 3(A) the effect of treatment with PMA and PMA + calcium ionophore A23187, hereinafter PMA-Ion, on the expression of mRNA of cox-2 is shown. In Figure 3(B) the inhibition by cyclosporin of the transcriptional induction of cox-2 is shown. In both cases, the result obtained for the non-inducible mRNAs of the cox-1 isoform and the glycerol-aldehyde dehydrogenase (GAPDH) by way of control is shown.
Figure 4 shows the result of the stimulation by PMA or by PMA+Ion of the luciferase activity in Jurkat cells transitorily transfected with the prom2-1906-LUC construct. As a control, it is checked that both the prom1-898-LUC construct and the empty plasmid PXP2 are not inducible.
Figure 5 shows the result of inhibition by cyclosporin A (CsA) of the stimulation caused by PMA-Ion of the construct prom2-1906-LUC in the transient transfection in Jurkat cells.
Figure 6 shows the results of an experiment of transitory transfection with the prom2-1906-LUC construct and treatment with dexamethasone.
Figure 7 shows the results of the luciferase activity of different clones obtained after stable transfection with the prom2-1906-LUC construct.
Figure 8 shows the results of inhibition by cyclosporin A (CsA) of the stimulation by PMA+Ion of the luciferase activity of the stable clones of Jurkat-1906LUC.
Figure 9 shows the results obtained for the inhibition by the glucocorticoide dexamethasone (Dex) on the stimulation by PMA+Ion of the luciferase activity of the stable clones of Jurkat-1906LUC.
Figure 10 shows the inhibition by Resveratrol (Res) of the induction of luciferase activity of the stable clones as a control of the assay system.

### DETAILED DESCRIPTION OF THE INVENTION

This invention provides a DNA construct (or recombinant DNA) that comprises the whole or part of a promoter sequence of the cyclooxygenase 2 (cox-2) gene, as defined in claim 1, and a reporter gene, operatively joined to each other, such that said promoter sequence of the cox-2 gene controls the expression of said reporter gene in response to a suitable stimulus.

The promoter sequence of the cox-2 gene may be of any origin, although preferably said sequence will proceed from the human cox-2 gene.

As a reporter gene, any reporter gene may be used that is able to produce an easily detectable signal, chosen from among those normally used in these types of transfection assays, for example, the chloranphenicol acetyl transferase (CAT) gene, the beta galactosidase (β-gal) gene, the luciferase gene, for example, from glow-worm or from *Renilla*. In a particular embodiment of this invention, said reporter gene is the luciferase gene of glow-worm because of its extreme sensitivity, speed and ease of use and low cost of the assay for its detection.

The invention also provides a vector, such as a plasmid or an expression vector, which contains the aforementioned DNA construct. In principle, any vector can be used that is suitable for inserting into said DNA construct. These vectors are useful for the transformation of cells.

The cell line provided by this invention comprises, and expresses in a stable fashion, said DNA construct that comprises all or part of a promoter sequence of the cox-2 gene and a reporter gene, operatively joined to each other, such that said promoter sequence of the cox-2 gene controls the expression of said reporter gene in response to a suitable stimulus.

The transformed cell line that contains the aforementioned DNA construct may originate from any suitable cell line able to express said DNA construct in a stable fashion, for example, a cell line of human origin such as a line of cells of the T-lymphocyte type, Hep-G2 cells derived from hepatocellular carcinoma, Hela cells derived from an adenocarcinoma of the cervix, cells of monocyte-macrophage type, for example, the lines U937 and THP-1, etc. In a particular embodiment of this invention, a Jurkat cell line has been selected (originally described by Schneider et al., 1977) as a representative example of a transformed cell line of the T-lymphocyte type as a model for studying the expression of a gene related to the immune response. In addition, said cell line is easy to grow and provides a high yield of cells per unit time and volume (ml) of culture.

The cell line provided by this invention can be used as an assay system in the search and identification (screening) of compounds that selectively inhibit the induction at a transcriptional level of cox-2 by different stimuli.

The cell line provided by this invention can be easily obtained using conventional procedures of Genetic Engineering, for example, by means of a process that comprises (i) the isolation of a promoter sequence of the cox-2 gene, (ii) the cloning of said sequence in a vector that contains the reporter gene, in a position in which said promoter sequence is able to control the expression of said reporter gene, and (iii) the transfection of a suitable cell line with said plasmid. In Example 3, there is described in detail a specific way for obtaining individual clones of transformed Jurkat cells which express the reporter gene (luciferase) in a stable fashion, denominated Jurkat-C3-1906LUC, Jurkat-F9-1906LUC and Jurkat-C7-1906LUC, in which the basal luciferase activity was determined and it was checked that the expression of the reporter gene (luciferase) was being induced in response to the same stimuli as the promoter of cox-2 as had been previously established with transitorily transfected cells.

The assay system (cell line) provided by this invention has been previously validated by the transitory transfection of the prom2-1906-LUC construct and the analysis of the luciferase activity under different stimuli and inhibitors. The results obtained were compared with a non-inducible control of a similar construct in which, instead of the promoter of cox-2, the promoter of the cox-1 isoform was put in place and with the empty vector pXP2. Under the same conditions, the behaviour of the endogenous cox-2 gene was also determined by experiments of RT-PCR in which the expression of mRNA is analysed. As a non-inducible control, the behaviour of the endogenous gene of the cox-1 isoform and the non-inducible gene of glycerol-aldehyde-phosphate dehydrogenase (GAPDH) was determined. The main treatments were activators such as phorbol ester PMA (10 ng/ml) and the combination of PMA and calcium ionophore A23187 [PMA+Ion] (1 µM). Treatment with drugs that inhibit the induction by PMA+Ion were carried out with dexamethasone (1 µM), and with cyclosporin A (100 ng/ml).

Example 2 includes some validation assays of the assay system provided by this invention in transitory transfection, as well as the expression of the endogenous genes in Jurkat cells. Also, in Examples 3 and 4, some assays are presented carried out with the stable clones obtained, with compounds that induce and inhibit the induction the cox-2 promoter.

The assay system provided by this invention is useful for the search and identification (screening) of compounds that selectively inhibit the induction at a transcriptional level of cox-2 by different stimuli. This type of selective inhibitor of cox-2 may have numerous potential therapeutic applications as the implications derived from the induction of cox-2 not only affect the inflammatory response, but also processes related to uncontrolled cellular proliferation and formation of tumours (for example, the appearance of adenomas, cancer of the colon and the development of polyps and angiogenesis, among others), with immunosuppressant actions and with neurodegenerative processes such as Alzheimer's disease. As a result, it might be supposed that the compounds that selectively inhibit the transcriptional induction of cox-2 may be useful as anti-inflammatory agents, as compounds able to attenuate uncontrolled cellular proliferation and/or the process of tumorigenesis, as immunosuppressants or as potential drugs with therapeutic properties in Alzheimer's disease.

The invention also provides an assay method for searching and identifying (screening) compounds that selectively inhibit the induction at a transcriptional level of cox-2 by a suitable stimulus (described in Example 4) which comprises bringing the cell line provided by this invention (assay system) into contact with the compound to be assayed, in other words, with the compound whose potential selective inhibitory activity of the induction at a transcriptional level of cox-2 it is wanted to test, in conditions that allow the transcription of cox-2, and detecting, and/or measuring, the signal indicative of the expression of activity due to the reporter gene. Alternatively, if so desired, the assay method object of this invention can be performed by bringing the cell line, the assay system and the compound that activates transcription induction of cox-2 into contact.

In the assay method provided by this invention, the regulation of the expression of the cox-2 gene is determined by the regulatory activity of its promoter, while the activity of the reporter gene provides an indirect measure of the activity of the cox-2 promoter in response to different agents.

The assay method for the search and identification of compounds that selectively inhibit the induction at a transcriptional level of cox-2 by an appropriate stimulus provided by this invention allows the selection of compounds that inhibit the production of cox-2 by means of a criterion based on the inhibition of the inducible activity of the cox-2 promoter, which will not select those compounds that inhibit the basal physiological activity of the production of cox-2.

The following examples serve to illustrate preferred embodiments of the invention, but they should not be considered as limiting the scope thereof.

### EXAMPLE 1

### Production of a DNA construct that comprises a promoter sequence of cox-2 and the luciferase gene

### 1.1 Cloning the promoter of cox-2

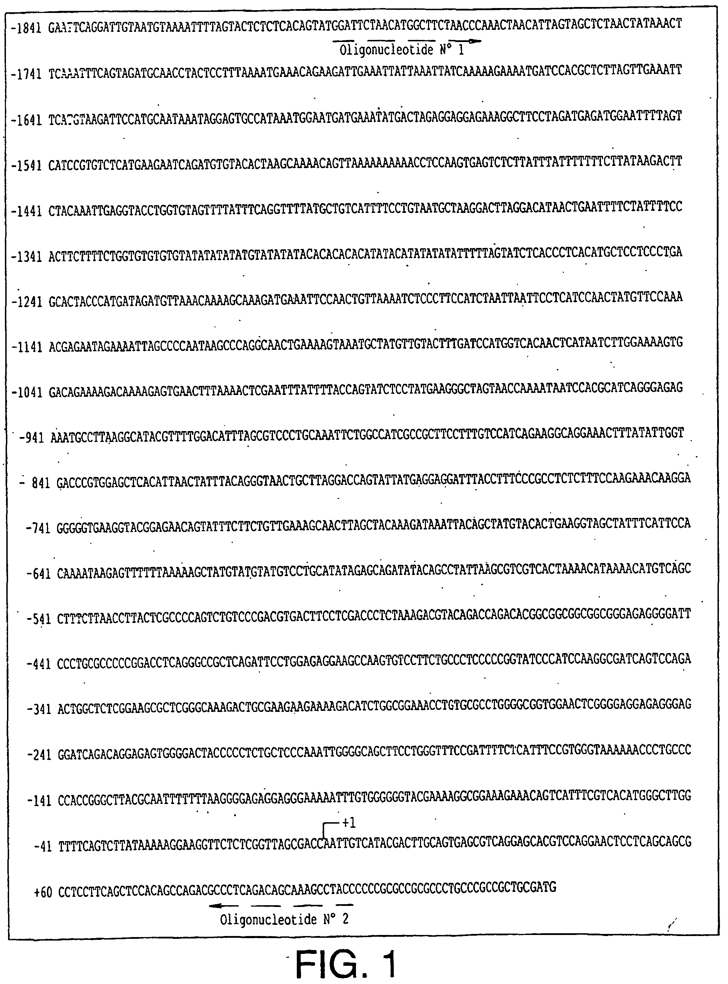

In the first place, the promoter sequence of the human cox-2 gene was cloned from the sequence described by [Tazawa et al., 1994] represented in Figure 1.

The technique of polymerase chain reaction (PCR) was used, with the initiating oligonucleatides or "primers" designed for selective amplification of the fragment of DNA corresponding to the promoter sequence of this gene. The template DNA used was genomic DNA from the Jurkat human lymphocyte cell line. The oligonucleotides used were those identified as SEC.ID.No.: 1 and SEC.ID.No.: 2 [see the section concerning the LIST OF SEQUENCES].

These oligonucleotides amplify a sequence that ranges from nucleotide -1796 to nucleotide +104 of the promoter zone of the cox-2 gene (see Figure 1). For the PCR reaction, the *Advantage cDNA PCR kit* [Clontech] was used with 30 cycles repeated every 45 seconds at 94°C and 3 minutes at 68° C in a PTC-200 thermocycler PTC-200 [MJ Research].

### 1.2 Construction of the expression vector

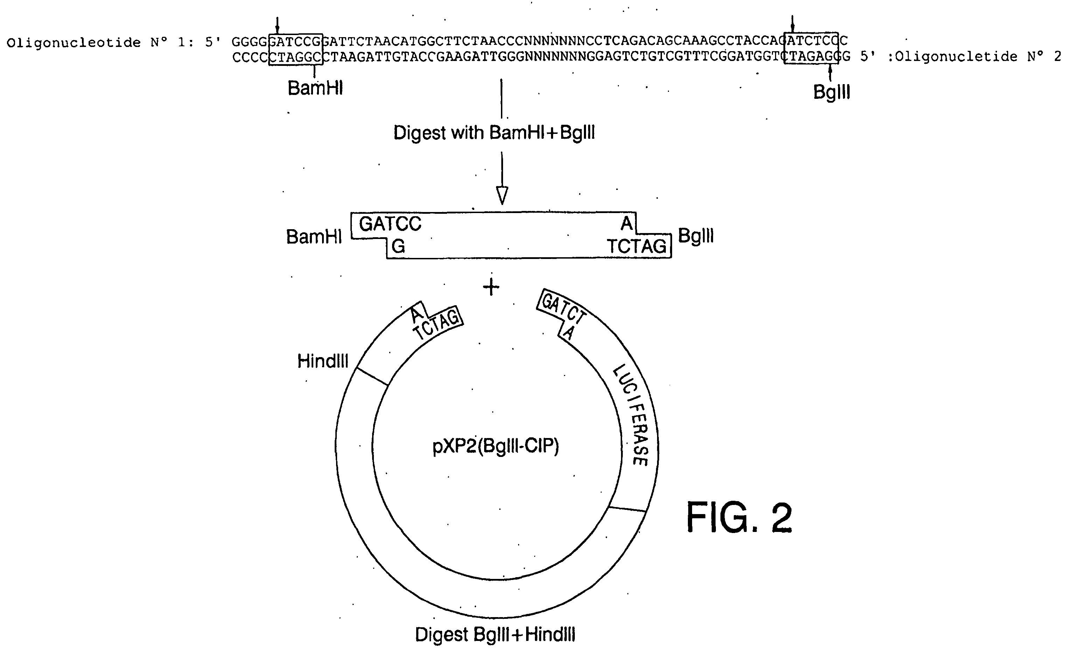

The fragments generated after amplification were subcloned into the plasmid pXP2 [Nordeen, 1988] which contains the sequence that codes for the luciferase gene which will be used as the reporter gene (see Figure 2). The oligonucleotides were designed such that at the 5' end they contain an additional recognition sequence for restriction enzymes. After amplification, double chain ends are generated that contain the restriction targets BamHI at the 5' end and BglII at the 3' end. The pXP2 vector contains a BglII target at the multiple cloning site, which generates ends compatible both with BglII and BamHI ends. After digestion of the insert obtained by PCR containing the promoting sequence with the BglII and BamHI enzymes and the pXp2 vector with BglII, the binding of these sequences was performed. In this fashion, the plasmid prom2-1906-LUC was obtained in which the sequence (-)1796-(+)104 of the cox-2 promoter is located in front of the luciferase gene, controlling its expression. This construction was sequenced to check the fidelity of the promoter sequence and to verify the cloning site.

### Example 2

### Experiments in analysis of the regulation of the activity of the promoter of cox-2 by means of experiments with RT-PCR and transitory transfection of the prom2-1906-LUC construct in the Jurkat cell line

In order to study the regulation of the expression of the endogenous cox-2 gene in the Jurkat cell line, the analysis of the expression of its mRNA was performed by experiments with RT-PCR. At the same time, in order to validate the prom2-1906-LUC construct and to check that the regulation of the cloned promoter is as expected, experiments were carried out of transitory transfection of the prom2-1906-LUC construct using the Jurkat cell line. In both experiments, the cells were treated with compounds that stimulate and inhibit the induction of the promoter of cox-2.

Treatment with activator compounds were carried out using the phorbol ester PMA (Phorbol 12-Myristate 13-Acetate) (10 ng/ml) (Sigma) and the combination of PMA and Calcium ionophore A23187 (1µM) (Sigma), hereinafter PMA+Ion.

Treatment with drugs that inhibit the induction by PMA+Ion were carried out with cyclosporin A (CsA) (100 ng/ml) or the synthetic glucocorticoide Dexamethasone (Dex) (1µM) (Sigma).

### 2.1 Regulation of the expression of cox-2 mRNA in Jurkat cells

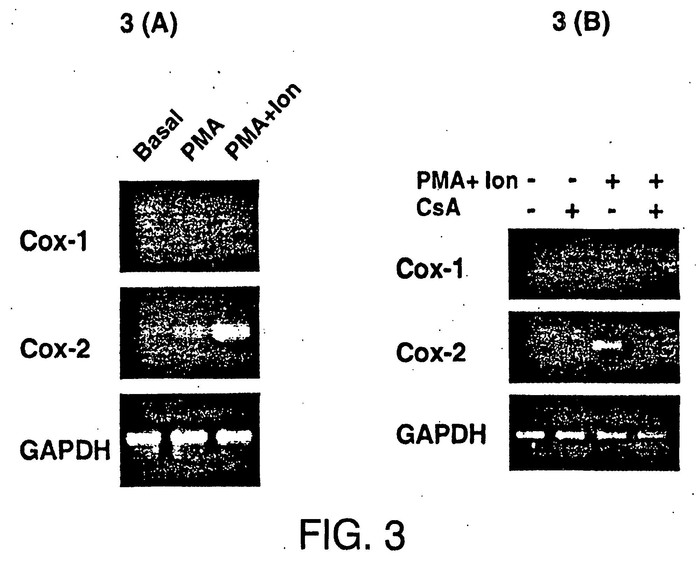

The results obtained from the analysis by RT-PCR of the expression of mRNA of cox-2 in Jurkat cells are presented in Figure 3, where the following can be observed:
a) treatment with PMA (10 ng/ml) produces a slight increase in the expression of the mRNA of cox-2, while the combination treatment with PMA+Ion leads to a greater increase in the expression of this gene at a transcriptional level [Figure 3(A)]; and
b) inhibition by CsA (100 ng/ml) of the transcriptional induction of cox-2 [Figure 3(B)].

As a control in both cases, the result obtained for the non-inducible mRNAs of the cox-1 isoform and for the glycerol aldehyde phosphate dehydrogenase (GAPDH) are shown.

### 2.2 Evaluation of the luciferase activity under different stimuli

The luciferase activity in Jurkat cells transitorily transfected with the prom2-1906-LUC construction using the Lipofectin agent [Life Technologies] has been analysed. The cells were stimulated with PMA (10 ng/m) (Sigma) and with the combination PMA+Ion (1 µM) (Sigma).

As a non-inducible control, a similar construct was used in which, instead of the cox-2 promoter, the promoter of the cox-1 isoform was put in place [prom1-898-LUC] and with the empty vector pXP2.

The luciferase activity was determined using the kit "Luciferase Assay System" [Promega], with 1x10⁶ cells which where lysed in 50 µl of lysis buffer. In the extracts obtained, the light emission produced was measured using a luminometer Monolight 2010 [Analytical Luminiscence Laboratory] with an automatic injection system of 100 µl of reagent.

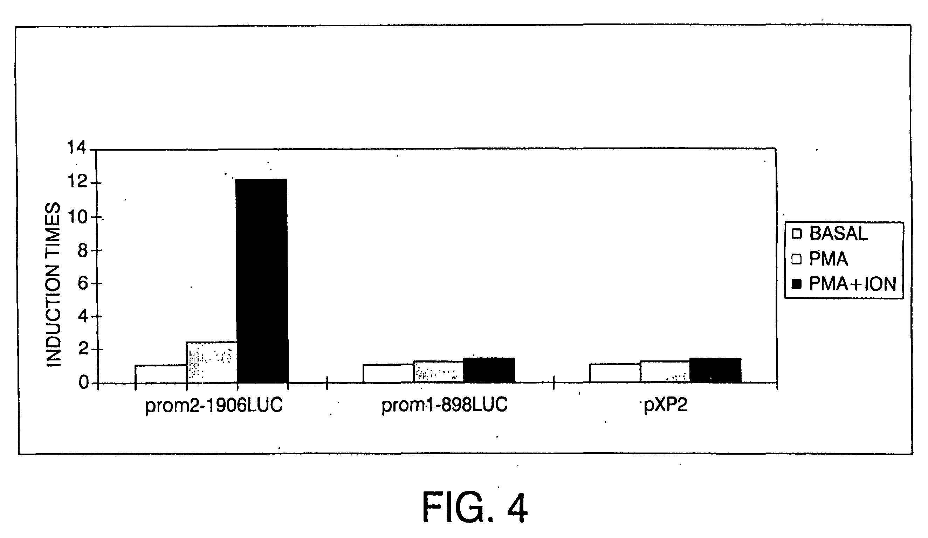

The results obtained are shown in Figure 4. As can be seen in said figure, the results obtained in this assay are comparable to those obtained in the analysis of mRNA of cox-2 [Example 2.1], in other words, treatment with PMA+Ion produces a greater increase in the number of times there is induction of the luciferase activity (approximately 12 times the basal value). These data show that the cloned promoter sequence behaves in a similar fashion to the endogenous gene. As a control, it is checked that neither the proml-898-LUC construct nor the empty plasmid pXP2 are inducible.

### 2.3 Evaluation of the luciferase activity under different inhibitors

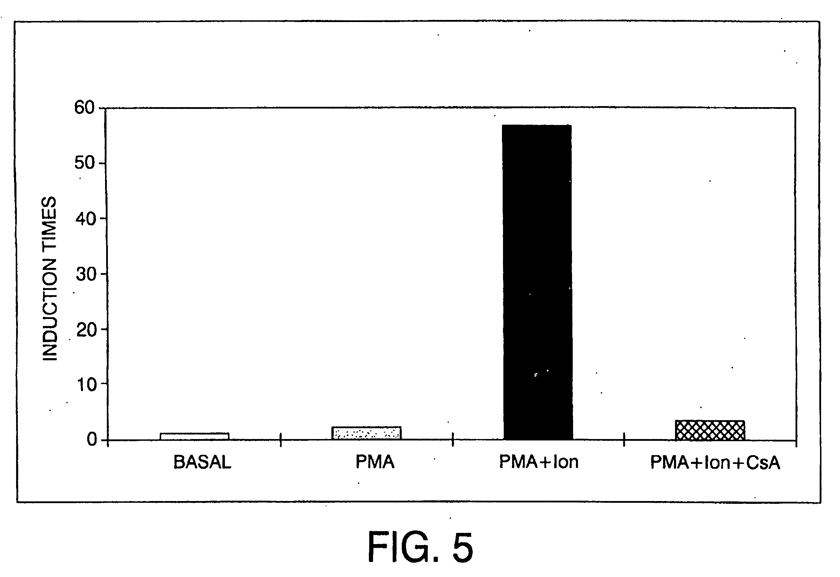

The inhibition of stimulation by PMA (10 ng/m) (Sigma) or PMA+Ion (1 µM) (Sigma) on the luciferase activity in Jurkat cells transitorily transfected with the prom2-1906-LUC construct has been investigated by using the immunosuppressant drug cyclosporin A (CsA) (100 ng/ml). The results obtained are shown in Figure 5 where it can been seen that treatment with CsA (100 ng/ml) reduces stimulation of the promoter of cox-2 in response to PMA+Ion to values similar to basal ones.

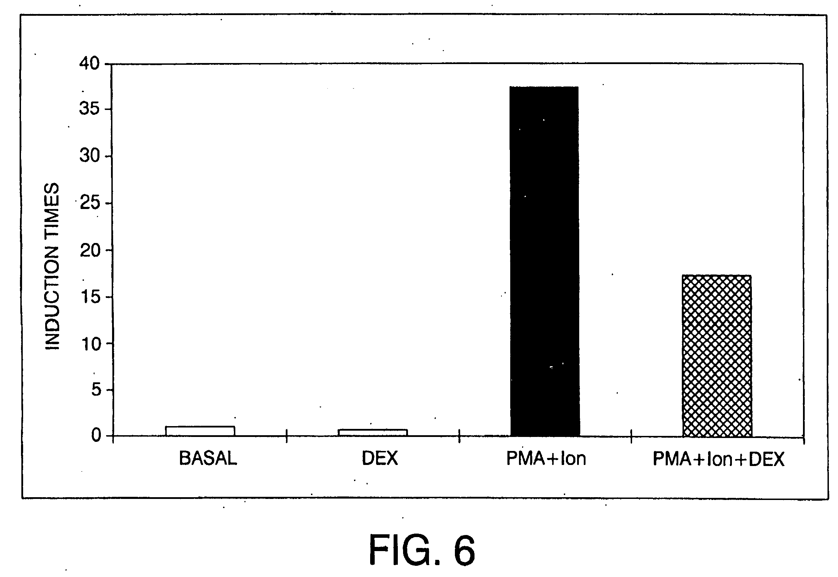

Similarly, the inhibition of stimulation by PMA+Ion (1 µM) (Sigma) on the luciferase activity has been analysed in Jurkat cells transitorily transfected with the prom2-1906 construct by means of the glucocorticoide dexamethasone (1 µM) (Sigma). The results obtained are shown in Figure 6 where it can be seen that treatment with dexamethasone (1 µM) (Dex) reduces stimulation of the promoter of cox-2 in response to PMA+Ion.

### Example 3

### Production of a cell line that stably expresses a DNA construct that comprises a promoter sequence of cox-2 and the luciferase gene.

For the creation of a cell line that stably expresses the prom2-1906-LUC construct, Jurkat cells were co-transfected with the vector prom2-1906-LUC and a vector denominated pcDNA3.1/Hygro (Invitrogen) which contains the gene for resistance to hygromycin. The transfection was carried out by means of the technique of electroporation in cuvettes of 0.4 cm (BioRad) with 15 x 10⁶ cells in 0.5 ml of complete medium [RMPI medium supplemented with 10% of foetal serum, L-glutamine 2mM and a mixture of antibiotics] (All these products were acquired from Life Technologies). The cells were incubated over ice for 10 minutes with 25 µg of plasmid prom2-1906-LUC and 5 µg of the vector pCDNA3.1./Hygro. After this period, the cells were electroporated in a Gene Pulser II (BioRad) apparatus at 1.500 µFaradays of capacitance and a current of 280 V. Next, the cells were incubated over ice for 10 minutes before adding 10 ml of complete medium. The cells were cultured in this medium in 75 cm² culture flasks (Nunc) for 48 hours in a cell incubator at 37° C with a humidity of 95% and 5% of CO₂. At this time, the medium was changed for complete medium with no antibiotics to which hygromycin (Boehringer Mannheim) was added at a concentration of 200 µg/ml. The cells were cultured in this medium for 30 days with successive changes of medium. During this period the resistant population that survived treatment with the selective antibiotic was selected, in other words, the cells stably transfected with the gene for resistance to the hygromycin antibiotic. In this population the expression of the luciferase gene was analysed in order to determine the presence of transfectants stable for the plasmid prom2-1906-LUC. For this, 1x10⁶ cells were lysed in 50 µl of lysis buffer (Promega) and with the extracts obtained, the luciferase activity was determined using the reagents contained in the kit of the "Luciferase Assay System" [Promega]. Measurement of the light produced was determined using a luminometer MonoLight 2010 (Analytical Luminiscence Laboratory) with a system of automatic injection of 100 µl of reagent.

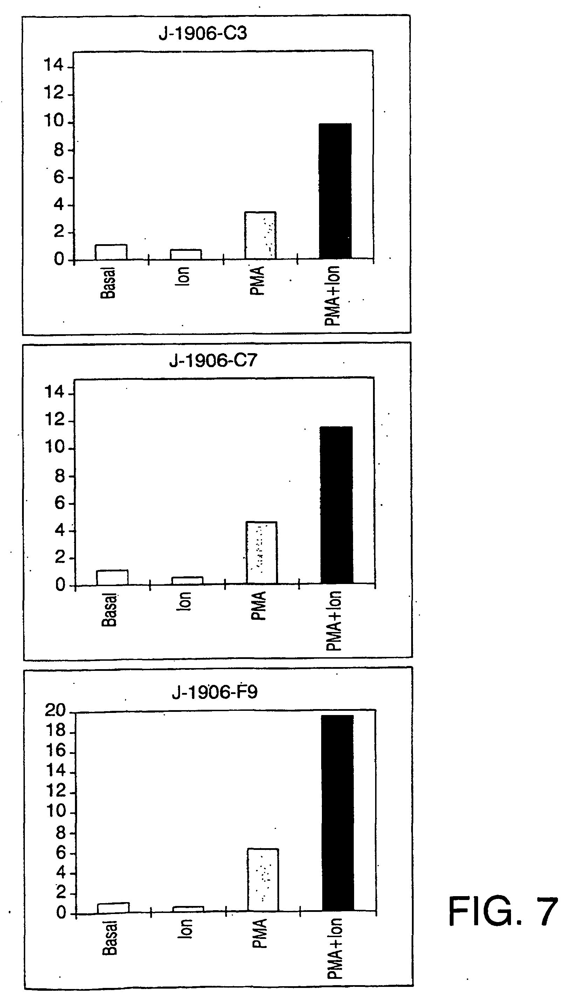

From this polyclonal population (Jurkat-pool-1906LUC) a limit dilution was performed on 96-well plates in complete medium with hygromycin in order to obtain individual clones that expressed the luciferase gene in a stable fashion. These clones were grown until obtaining at least 1x10⁶ cells with which measurement of the luciferase activity could be performed as described earlier. In this way, three individual clones were obtained denominated Jurkat-C3-1906LUC, Jurkat-F9-1906LUC and Jurkat-C7-1906LUC. In these clones the basal luciferase activity was determined in RLU (relative luminescence units) and it was checked that the expression of the luciferase reporter was being induced in response to the same stimuli as the promoter of cox-2 as had been established previously with cells transitorily transfected (see Figure 7). In the three clones, the basal values of luciferase activity increase from 3 to 6 times with a treatment of 6 hours with the phorbol ester PMA and up to 10 - 20 times with a combined treatment PMA+Ion, in a similar fashion to the results obtained in the cells transfected transitorily.

### Example 4

### Establishment of an assay system for compounds that regulate the expression of the gene cox-2 in the clones of the cell line that stably expresses a DNA construct that comprises a promoter sequence of cox-2 and the luciferase gene.

The clones were cultured on plates of 96-wells at a density of 1x10⁵ cells in 200 µl of RPMI medium supplemented with 2% of foetal serum, L-Glutamine 2 mM and a mixture of antibiotics. The cells were treated for 6 hours with different concentrations of compounds whose activity it was hoped to analyse. In the case of the assay of the activity of these compounds on the induction of activity of the cox-2 promoter, the cells were treated with PMA+Ion for 5 hours, after 1 hour of pre-treatment with the compound to test. After this period, the cells were lysed in 50 µl of lysis buffer and their luciferase activity determined using 20 µl in a luminometer as was described in Example 2.2. Next, some results obtained are shown with compounds previously described as inhibitors of the stimulation of the cox-2 promoter.

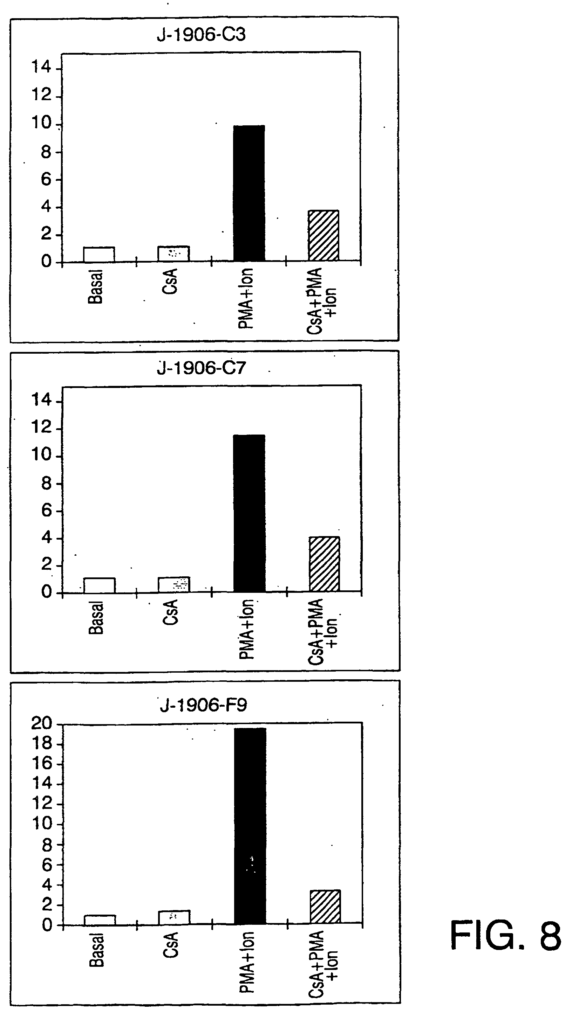

Figure 8 shows the results obtained with the compound cyclosporin A (CsA) which produces an inhibition of the stimulation obtained with PMA+Ion in stable Jurkat clones, in a similar fashion to that already described (Iñiguez et al., 1998), and that observed in the transitory transfections illustrated in Example 2.3.

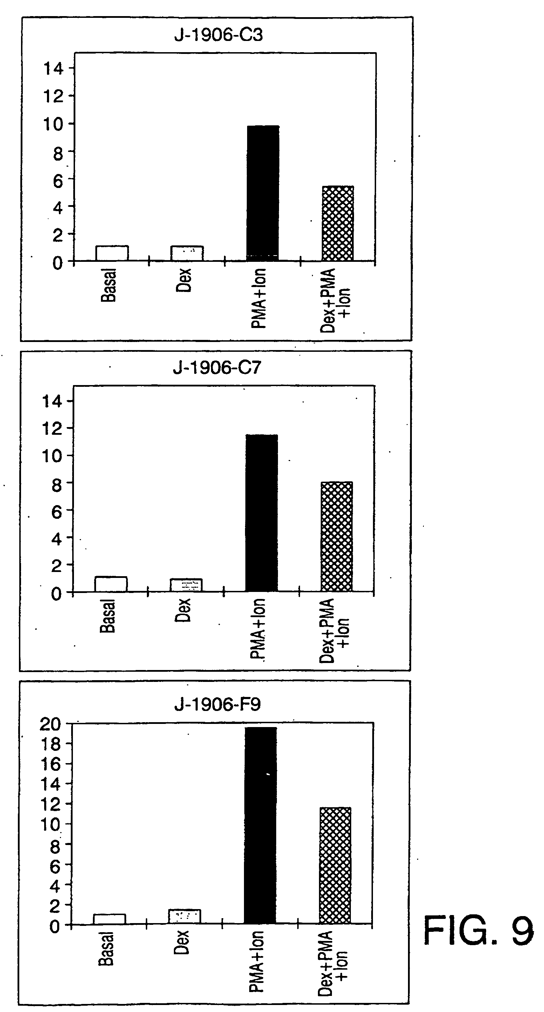

Figure 9 shows the results obtained with the compound Dexamethasone (Dex), which, like glucocorticoide and anti-inflammatories, produces an inhibition of the stimulation obtained with PMA+Ion in the stable Jurkat clones, corresponding to that already described (Smith and DeWitt, 1996) and that observed previously in the transitory transfections of Example 2.3.

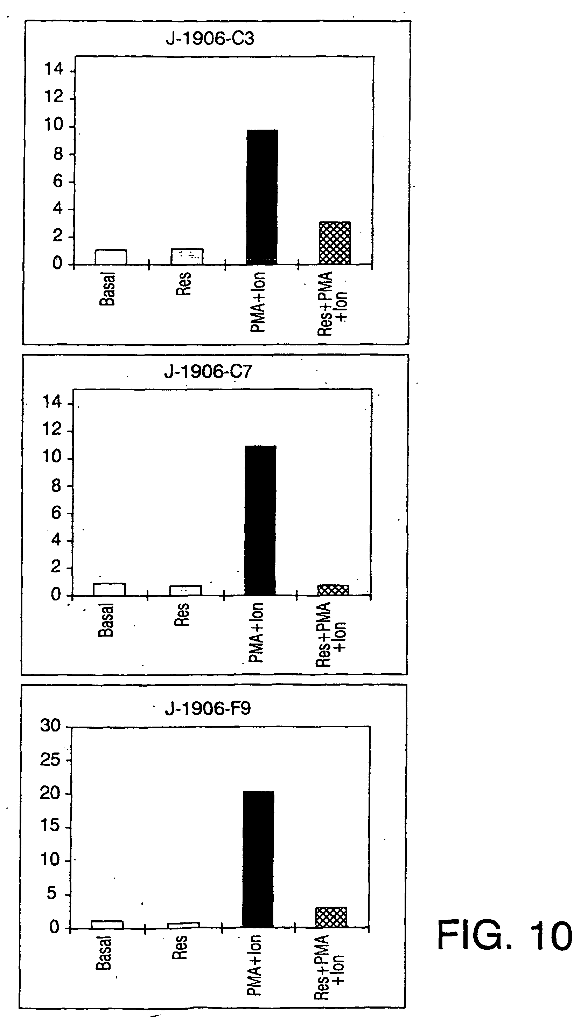

Figure 10 shows the results obtained with the compound Resveratrol (Res), recently described as an inhibitor of the stimulation by PMA of the gene cox-2 (Subbaramiah, et al., 1998). This compound produces an inhibition of the stimulation obtained with PMA+Ion om the stable Jurkat clones.

The validity of the assay system is thus demonstrated due to its similar behaviour for the clones obtained, for the transitory transfections and the results obtained with endogenous mRNA. With the use of compounds whose activity on the cox-2 promoter is known, whether they be stimulatory or inhibitory, it is established that it is possible to detect both positively and negatively the basal or induced expression of the cox-2 gene with the assay system developed in present invention.

### DEPOSIT OF BIOLOGICAL MATERIAL

A sample of a Jurkat cell line, denominated J-1906-F9, that stably expresses a DNA construct that comprises a promoter sequence of the cox-2 gene and the luciferase gene, has been deposited in the European Collection of Animal Cell Cultures (ECACC) [Salisbury, United Kingdom] on the 24 March 1999 and has been assigned the access number ECACC 99032405.

A sample of the plasmid prom2-1906-LUC, inserted into *Escherichia coli* DH5, denominated DH5 prom2-1906-LUC, has been deposited in the Spanish Collection of Culture Types (CECT) [Burjassot, Valencia] on the 24 March 1999 and has been assigned the access number CECT 5145.

### REFERENCES

Berg, J., Christoph, T., Widerna, M., and Bodenteich, A. 1997. Isoenzyme-specific cyclooxygenase inhibitors: a whole cell assay system using the human erythroleukemic cell line HEL and the human monocytic cell line Mono Mac 6. *J. Pharmacol. Toxic Methods.* 37: 179-86.

Brideau, C., Kargman, S., Liu S., Dallob A.L., Enrich E.W., Rodger, I.W., And Chan C.C. 1996. A human whole cell assay for clinical evaluation of biochemical efficacy of cyclooxygenase inhibitors. *Inflamm. Res*. 45: 68-74.

Cromlish W.A., and Kennedy, B.P. 1996. Selective inhibition of cyclooxygenase-1 and -2 using intact insect cell assays. *Biochem. Pharmacol.* 52: 1777-85.

Famaey, J.P. 1997. In vitro and in vivo pharmacological evidence of selective cyclooxygenase-2 inhibition by nimesulide: an overview. *Inflamm. Res.* 46: 437-446.

Griswold, D.E., and Adams, J.L. 1996. Constitutive cyclooxygenase (cox-1) and inducible cyclooxygenase (cox-2): Rationale for selective inhibition and progress to date. *Med. Res. Rev.* 16: 181.

Hall, V.C., and Wolf, R.E. 1997. Effect of Tenidap and nonsteroidal antiinflammatory drugs on the response of cultured human T cells to interleukin 2 in rheumatoid arthritis. *J. Rheumatol*. 24:1467.

Iñiguez, M.A., Punzón, C., and Fresno, M. 1998. Induction of cyclooxygenase-2 on activated T lymphocytes; regulation of T cell activation by cyclooxygenase-2 inhibitors. Sent to publish.

Jouzeau, J.Y., Terlain, B., Abid, A., Nedelec, E., and Netter, P. 1997. Cyclooxygenase isoenzimes. How recent findings affect thinking about nonsteroidal anti-inflammatory drugs. *Drugs* 53: 563.

Lora, M., Morisset, S., Menard, H.A., Leduc, R., and de Brum-Fernandes, A.J. 1997. Expression of recombinant human cyclooxygenase isoenzymes in transfected COS-7 cells in vitro and inhibition by tenoxicam, indomethacin and aspirin. *Prostaglandins Leukot. Essent. Fatty Acids.* 56: 361-7.

Nordeen, S.K. 1988. Luciferase reporter gene vectors for analysis of promoters and enhancers. *Biotechniques 6*: 454-457.

Noreen, Y., Ringbom, T., Perera, P., Danielson, H., And Bohlin, L. 1998. Development of a radiochemical cyclooxygenase-1 and -2 in vitro assay for identification of natural products as inhibitors of prostaglandin biosynthesis. *J. Nat. Prod.* 61: 2-7.

O'Neill, G.P., Kennedy, B.P. Mancini, J.A., Kargman, S., Ouellet, M., Yergey, J., Falgueyret, J.P., Cromlish, W.A., Payett., P., Chan, C.C. et al. 1995. Selective inhibitors of Cox-2. *Agents Actions Suppl*. 46: 159-68.

Pasinetti, G.M. 1998. Cyclooxygenase and inflammation in Alzheimer's disease. Experimental approaches and clinical interventions. J. Neurosci. Res. 54: 1-6.

Schneider U., Schwenk, H.U., and Bornkamm, G. 1977. Characterization of EBV-genome negative "null" and "T" cell lines derived from children with acute lymphoblastic leukemia and leukemic transformed non-Hodkin lymphoma. Int. J. Cancer, 19:521-6.

Sheng, H., Shao, J., Kirkland, S.C., Isakson, P., Coffey, R.J., Morrow, J., Beauchamp, R.D., and DuBois, R.N. 1997. Inhibition of human colon cancer cell growth by selective inhibition of cyclooxygenase-2. *J. Clin. Invest*. 99: 2254-2259.

Shiff, S.J., Koutsos, M.I., Qiao, L., and Rigas, B. 1996. Nonsteroidal antiinflammatory drugs inhibit the proliferation of colon adenocarcinoma cells: effect on cell cycle and apoptosis. *Exp. Cell Res.* 222, 179-188.

Smith, W..L., and DeWitt, D.L. 1996. Prostaglandin Endoperoxide H Synthase-1 and -2. *Adv. Immunol.* 62:167.

Subbaramiah, K., Chung, W.J., Michualart, P., Telang, N., Tanabe, T., Inoue, H., Jang, M., Pezzuto, J.M., and Dannenberg, A.J.1998. Resveratrol inhibits cyclooxygenase-2 transcription and activity in phorbolester treated human mammary epithelial cells. *J. Biol. Chem*. 273: 21875-882.

Tao, X., Schulze-Koops, H., Ma, L., Cai, J., Mao, Y., and Lipsky, P.E. 1998. Effects of Triptrygium wilfordii hook extracts on induction of cyclooxygenase 2 activity and prostaglandin E2 production. *Arthritis Rheum*. 41: 130-8.

Tazawa, R., Xu, X.M., Wu, K.K., and Wang, L.H. 1994. Characterization of the genomic structure, chromosomal location and promoter of human prostaglandin H synthase-2 gene. *Biochem. Biophys. Res. Comm*. 203:190-194.

Tsujii M., Kawano, S., Tsuji, S., Sawaoka, H., Matsatsugu, H., and DuBois, R.N. 1998. Cyclooxygenase regulates angiogenesis induced by colon cancer cells. *Cell* 93: 705-716.

Tsujii, M., Kawano, S., and DuBois, R.N. 1997. Cyclooxygenase-2 expression in human colon cancer cells increases metastatic potential. *Proc. Natl. Acad. Sci. USA* 94: 3336-40.

Zhou, L., Ritchie, D., Wang, E.Y., Barbone, A.G., Argentier D., And Lau, C.Y. 1994. Tepoxalin, a novel immunosuppressive agent with a different mechanism of action from cyclosporin A. *J. Immunol*. 153:5026.

### LIST OF SEQUENCES

(1) GENERAL INFORMATION:
   (i) APPLICANT:
      (A) NAME: Laboratorios del Dr. Esteve, S.A.
      (B) ADDRESS: Avda. Mare de Deu de Montserrat, 221
      (C) CITY: Barcelona
      (E) COUNTRY: Spain
      (F) POSTCODE: 08041
      (G) TELEPHONE: 93 446 60 00
      (H) FAX: 93 450 32 02
   (ii) TITLE OF THE INVENTION:
      CELL LINE THAT COMPRISES THE PROMOTER OF CYCLOOXYGENASE-2 (COX-2) AND A REPORTER GENE, AND USE THEREOF IN THE SEARCH FOR SELECTIVE INHIBITORS OF THE TRANSCRIPTIONAL INDUCTION OF COX-2
   (iii) NUMBER OF SEQUENCES: 2
   (iv) COMPUTER READABLE FORM:
      (A) MEDIUM: Diskette
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION ON THE IDENTIFIED SEQUENCE No. [SEC.ID.No.]: 1:
   (i) CHARACTERISTICS OF THE SEQUENCE:
      (A) LENGTH: 33 nucleotides
      (B) TYPE: nucleic acid
      (C) NO OF CHAINS: single chain
      (D) TOPOLOGY: linear
   (ii) TYPE OF MOLECULE: DNA
   (xi) DESCRIPTION OF THE SEQUENCE: SEC. ID. No.: 1: GGGGGATCCG GATTCTAACA TGGCTTCTAA CCC 33
(2) INFORMATION ON THE IDENTIFIED SEQUENCE No.: 2:
   (i) CHARACTERISTICS OF THE SEQUENCE:
      (A) LENGTH: 30 nucleotides
      (B) TYPE: nucleic acid
      (C) NO OF CHAINS: single chain
      (D) TOPOLOGY: linear
   (ii) TYPE OF MOLECULE: DNA
   (xi) DESCRIPTION OF THE SEQUENCE: SEC. ID. No.: 2:

GGGAGATCTG GTAGGCTTTG CTGTCTGAGG 30

## Claims

1. A DNA construct that comprises the sequence lying between the nucleotide - 1796 and the nucleotide + 104 of the promoter of human cyclooxygenase 2 (cox-2) gene and a reporter gene, operatively joined to each other, such that said promoter sequence of the cox-2 gene controls the expression of said reporter gene in response to a suitable stimulus.

2. A construct according to claim 1, in which said reporter gene is selected from the luciferase gene, the chloramphenicol acetyltransferase gene and the gene of beta galactosidase.

3. A vector that comprises a DNA construct according to any of claims 1 to 2.

4. A cell line that comprises.a construct according to any of claims 1 to 2 or transformed with a vector according to claim 3.

5. A cell line according to claim 4, in which said cell line is derived from a cell line of human origin.

6. A cell line according to claim 5, in which said cell line of human origin is a line of Jurkat cells.

7. A cell line according to claims 4 to 6 which expresses in stable fashion the DNA construct of claims 1 to 2.

8. An assay method for the search for compounds that selectively inhibit the induction at a transcriptional level of cyclooxygenase-2 by a suitable stimulus, that comprises bringing a cell line according to any of claims 5 to 7, into contact with a compound whose potential selective inhibitory activity of induction at a transcriptional level of cox-2 it is wanted to assay, in conditions that allow the transcription of cox-2, and detecting, and/or measuring, the signal indicative of the expression of activity due to the reporter gene.

## Patentansprüche

1. Ein DNA-Konstrukt, umfassend die zwischen dem Nukleotid -1796 und dem Nukleotid +104 des Promotors des Human-Cyclooxygenase 2 (Cox-2) Gens liegende Sequenz und ein Reportergen, die operativ miteinander verbunden sind, so dass diese Promotorsequenz des Cox-2 Gens die Expression des Reportergens als Antwort auf einen geeigneten Stimulus regelt.

2. Ein Konstrukt nach Anspruch 1, in dem das Reportergen ausgewählt ist aus dem Luciferasegen, dem Chloramphenicol-Acetyltransferasegen und dem Gen der beta-Galactosidase.

3. Ein Vektor, der ein DNA-Konstrukt nach einem der Ansprüche 1 bis 2 umfasst.

4. Eine Zelllinie, die ein Konstrukt nach einem der Ansprüche 1 bis 2 umfasst oder mit dem Vektor nach Anspruch 3 transformiert ist.

5. Eine Zelllinie nach Anspruch 4, die abgeleitet ist von einer Zelllinie menschlicher Herkunft.

6. Eine Zelllinie nach Anspruch 5, wobei die Zelllinie menschlicher Herkunft eine Jurkat-Zelllinie ist.

7. Eine Zelllinie nach einem der Ansprüche 4 bis 6, die in stabiler Weise das DNA-Konstrukt der Ansprüche 1 bis 2 exprimiert.

8. Ein Testverfahren zur Suche nach Verbindungen, die selektiv die Induktion von Cyclooxygenase-2 auf Transskriptionsebene durch einen geeigneten Stimulus hemmen, umfassend das in Kontakt bringen einer Zelllinie nach einem der Ansprüche 5 bis 7 mit einer Verbindung, deren potentielle selektiv inhibierende Wirkung der Induktion von Cox-2 auf Transskriptionsebene untersucht werden soll unter Bedingungen, die die Transskription von Cox-2 erlauben, und das Nachweisen und/oder Messen des die Expression anzeigenden Signals auf Grund der Aktivität des Reportergens.

## Revendications

1. Produit de recombinaison d'ADN qui comprend la séquence placée entre le nucléotide - 1796 et le nucléotide + 104 du promoteur du gène de la cyclo-oxygénase 2 (COX-2) humain et un gène rapporteur, reliés l'un à l'autre de manière fonctionnelle, de telle sorte que la séquence promoteur du gène de la COX-2 commande l'expression du gène rapporteur en réponse à un stimulus adapté.

2. Produit de recombinaison selon la revendication 1, dans lequel le gène rapporteur est choisi parmi le gène de la luciférase, le gène de la chloramphénicol acétyltransférase et le gène de la bêta-galactosidase.

3. Vecteur qui comprend un produit de recombinaison d'ADN selon l'une quelconque des revendications 1 à 2.

4. Lignée cellulaire qui comprend un produit de recombinaison selon l'une quelconque des revendications 1 à 2, ou transformée par un vecteur selon la revendication 3.

5. Lignée cellulaire selon la revendication 4, **caractérisée en ce que** la lignée cellulaire est dérivée d'une lignée cellulaire d'origine humaine.

6. Lignée cellulaire selon la revendication 5, **caractérisée en ce que** la lignée cellulaire d'origine humaine est une lignée de cellules Jurkat.

7. Lignée cellulaire selon les revendications 4 à 6, **caractérisée en ce qu'** elle exprime de façon stable le produit de recombinaison d'ADN des revendications 1 à 2.

8. Procédé d'analyse pour la recherche de composés qui inhibent sélectivement l'induction à un niveau transcriptionnel de la cyclo-oxygénase 2 par un stimulus adapté, **caractérisé en ce qu'** on amène une lignée cellulaire, selon l'une quelconque des revendications 5 à 7, en contact avec un composé dont on veut analyser l'activité inhibitrice sélective potentielle de l'induction à un niveau transcriptionnel de la COX-2, dans des conditions qui permettent la transcription de la COX-2, et de détecter et/ou de mesurer le signal indicateur de l'expression de l'activité due au gène rapporteur.
